# EUROPEAN PATENT APPLICATION

(11) **EP 4 390 950 A1**
(43) Date of publication of application: **26.06.2024**
(21) Application number: 22214852.0
(22) Date of filing: 20.12.2022
(51) Int. Cl.: G16H 20/00, G16H 50/20

(54) **COMPUTER-IMPLEMENTED METHOD FOR DETERMINING PLANNING DATA FOR A SURGICAL PROCESS OF A SUBJECT**

(71) Applicant: Koninklijke Philips N.V., 5656 AG Eindhoven (NL)
(72) Inventor: PREVRHAL, Sven Peter, Eindhoven (NL); SCHMIDT-RICHBERG, Alexander, Eindhoven (NL); CAROLUS, Heike, 5656AG Eindhoven (NL); KLINDER, Tobias, Eindhoven (NL); STROOSMA, Otto Bernardus, Eindhoven (NL)
(74) Representative: Philips Intellectual Property & Standards

(57) **Abstract**

Computer-implemented method for determining planning data for a surgical process of a subject in a urology procedure, comprising: providing, by a processor, a prediction model trained to predict planning data based on at least one of historical patient data, historical task data, historical planning data, current patient data and current task data (S 100); obtaining, by the processor, at least one of: current patient data and current task data (S200); inputting the at least one of the current patient data and current task data to the prediction model in order to determine planning data (S300); providing a user recommendation on planning of a urology procedure planning, such as a PCNL procedure, by the processor, based on the determined planning data (S400).

## Description

### FIELD OF THE INVENTION

The invention relates to a computer-implemented method for determining planning data for a surgical process of a subject, to a device for determining planning data for a surgical process of a subject and to a computer program.

### BACKGROUND OF THE INVENTION

Medical procedures such as a surgery for example percutaneous nephrolithotomy (PCNL) are difficult to plan, as there is a plurality of influencing factors. However, the quality and the efficiency of such procedures depend on reliable planning data.

It has now become apparent that there is a further need to provide a method for determining planning data.

These and other objects, which become apparent upon reading the following description, are solved by the subject matter of the independent claims. The invention provides a method, a device and a computer program. The dependent claims refer to preferred embodiments of the invention.

### SUMMARY OF THE INVENTION

In view of the above, it is an object of the present invention to provide a method that allows for improving the determining of planning data for a surgical process of a subject, particularly a human being.

The inventors have realized that some medical procedures, such as urological surgeries (e.g. percutaneous nephrolithotomy PCNL: minimally invasive procedure to destroy and remove kidney stones from the kidney by a small puncture wound through the skin) have a high complication rate. Therefore, planning PCNL procedures can be challenging with respect to duration, required instruments, expertise level and personnel, including the personnel from other departments, including personnel that should be on standby.

These and other objects, which become apparent upon reading the following description, are solved by the subject matter of the independent claims. The invention provides a method, a device and a computer program. The dependent claims refer to preferred embodiments of the invention.

Considering the clinical complexities (e.g., variety of different patient factors to consider), the clinical workflows complexities, it is a difficult to plan for surgical procedures. For example, in PCNL, obesity of a patient, proximity of the calyx chosen for the initial puncture to organs at risk and artery, as well as the size of the stone and related risks of obstructing the ureter with stone fragments and the composition of the stone is a deciding factor in procedure duration and choice of a sectioning device. This makes every case unique with reference to the calyx and the position of the at least one stone. In some of the cases, the entire procedure has to be rescheduled due to insufficient planning data or some new factors, which were not taken into account.

In one aspect of the present disclosure, a computer-implemented method for determining planning data for a surgical process of a subject in a urology procedure, comprising:
providing, by a processor, a prediction model trained to predict planning data based on at least one of: historical patient data, historical task data, historical planning data, current patient data and current task data (S100);
obtaining, by the processor, at least one of: current patient data and current task data (S200);
inputting the at least one of the current patient data and current task data to the prediction model in order to determine planning data (S300);
providing a user recommendation on planning of a urology procedure planning, such as a PCNL procedure, by the processor, based on the determined planning data (S400).

The term "subject" is to be understood broadly in the present case and may comprise any humans and any animals.

The term "planning data", as used herein, is to be understood broadly and may relate to any data necessary or useful to plan a medical procedure such as an urology surgery. The planning data may comprise at least one of: staffing of personnel, equipment availability, room availability, room preparation, patient data, a planned and/or previous access plan of a PCNL needle to a region in a subject.

The term "surgical process", as used herein, is to be understood broadly and may relate to any surgical process carried out on a human or an animal. The surgical process may be for example, a urology procedure, such as a PCNL procedure.

The term "prediction model", as used herein, is to be understood broadly and may relate to any prediction model based on a machine learning algorithm or an artificial intelligence algorithm configured to be trained to predict planning data based on historical patient data, historical task data, historical planning data, current patient data and current task data. The prediction model may be trained with training data in order to predict planning data.

The machine learning model according to the present invention preferably may comprise at least one of: decision trees, naive bayes classifications, nearest neighbours, neural networks, convolutional neural networks (CNN), generative adversarial networks (GAN), multi-conditional GAN's, support vector machines, linear regression, logistic regression, random forest and/or gradient boosting algorithms.

The term "historical patient data", as used herein, is to be understood broadly and may relate to any patient data available from historical surgical processes. It is to be understood that historical surgical processes refer to surgical procedures, and associated patient data, of other patients, that may be used for comparison and planning for the surgical procedure of the current patient. Hence, the historical patient data may be received from a real surgical process that was carried out in the past. Many patient-specific data can be extracted from the imaging data, such as computed tomography (CT), ultrasound (US), fused US/MRI (magnetic resonance imaging) images, as well as patient metadata (e.g., age, body circumference resp. obesity, Body Mass Index, BMI, and others), which may come from a connected Enterprise Imaging System, Radiology Information System, Hospital Information system, Electronic Health record system, Electronic Medical system, and any combination of these. In some embodiments the information may come from the same hospital. In some embodiments, the information may come from different hospitals. The user, via a suitable user interface, may request the needed historical patient data from at least one system, such as the Electronic Health Record (EHR), system.

The term "historical task data", as used herein, is to be understood broadly and may relate to any task data available from historical surgical processes. That means that the historical task data may be received from a real surgical process that was carried out in the past. The historical task data may comprise data in relation to the clinical procedure/manipulation that was performed on the patient, such as the object to be treated, the clinical procedure code, etc.

The term "historical planning data", as used herein, is to be understood broadly and may relate to any planning data available from historical surgical processes. The historical planning data may comprise initial planning data and realized planning data. That means that the historical planning data may be received from a real surgical process that was carried out in the past. It should be noted that historical planning data were derived among others from historical task data. Hence, the historical task data may be at least a part of the historical planning data.

The term "current patient data", as used herein, is to be understood broadly and may relate to any patient data that is available for a future surgical process on a subject to be treated. The current patient data may comprise age, BMI or the like. This data may comprise historical data of the same patient. The difference between the "historical patient data" and "current patient data", is that the historical patient data may comprise data from other patients, wherein the current patient data may also comprise the historical information from the current patient.

The term "current task data", as used herein, is to be understood broadly and may relate to any task data that is available for a future surgical process on a subject to be treated. The current task data may comprise the object to be treated, a manner of treatment and a position of the object in subject. The term "current", as used herein, refers to the present or the future in comparison to historical that refers to the past.

The term "providing", as used herein, is to be understood broadly and may relate to any way of presenting data. "Providing" may comprise presenting the planning data and/or the user recommendation on a screen, Graphical User Interface (GUI) on a dashboard, in a data file, in a table or the like. In some embodiments, the user through e.g. the GUI, can add additional entries, such as patient's daily weight.

In other words, the current invention may relate to a use of a trained model to predict planning data for a current surgical process, wherein the model is trained with data on past surgical processes of other patients, as well as procedures in relation to the current patients. In some embodiments, the trained model may search for similar patients based on the similarity of parameters, such as the comparing the CT images of the patients. The combination of these data points may predict optimally the characteristics of a current planned surgical process as the model interpolates between the data points. For example, the prediction may be provided in a sense of "urologist who were faced with a similar task used the following equipment, staff and took 125 minutes to complete the surgical process". The prediction may be accompanied by diagrams, images, figures, tables, etc. This may be advantageous in terms of efficiency, quality, reduction of complexity, reduction of mistakes.

It should be clear to the skilled reader that the method is not carried out during a surgical process and does not interact with the subject to be treated.

Percutaneous nephrolithotomy (PCNL) is a minimally-invasive procedure to destroy and remove kidney stones from the kidney by a small puncture wound (up to about 1 cm) through the skin. Alternative to shock wave lithotripsy or ureteroscopy, PCNL is suitable for larger stones or stones of complicated shape (staghom stones) and is usually performed under general or spinal anaesthesia in the operating theatre either by a urologist or combined urologist-radiologist team.

PCNL is usually planned on the image data available from a diagnostic contrast CT scan and performed with the patient in prone position. Through the skin incision and under imaging guidance either by x-ray fluoroscopy or ultrasound, a hollow nephrolithotomy needle is advanced to the calyx deemed suitable to reach the stone on the CT images and a guide wire is inserted. Then, several dilating sheaths are inserted over the wire until the opening is large enough for the nephroscope to be passed and the stones to be taken out. Larger stones may need to be sectioned first using laser or cutter techniques.

Placing the guide wire for stone access is regarded as the more delicate part of the procedure and frequently performed by an interventional radiologist, sometimes even in a separate pre-procedure, rather than by the urologist, because the access path is close to sensitive structures such as vasculature, pleura, spleen, colon, liver, which can be accidentally punctured while establishing access to the calyx due to inaccurate needle placement and insertion.

While PCNL is typically well tolerated, complications are relatively frequent and intra-procedurally include ureteral stone (stone fragment sliding into the ureteral), failure of complete stone removal, perforation of the urinary collecting system, vascular injury as well as colonic perforation or pleural injury. Staghorn renal stones are challenging cases, requiring careful preoperative evaluation and close follow-up to avoid stone recurrence.

Because of their relatively high complication rate, planning PCNL procedures can be challenging with respect to duration, required instruments, expertise level and personnel, including personnel that should be on standby. Factors that need to be considered are patient- or stone-related, including obesity, proximity of the calyx chosen for the initial puncture to organs at risk and artery as well as the size of the stone(s) and related risk of obstructing the ureter with stone fragments and the composition of the stone which is a deciding factor in procedure duration and choice of sectioning device. This makes every case unique with reference to calyx anatomy and the position of stone or stones.

For instance, obese patients present several technical challenges including anaesthesia, patient positioning, imaging for access, longer skin-to-collecting-system distances, and nephrostomy tube dislodgement.

The invention proposes to use an AI-based algorithm, e.g. a machine learning algorithm, to provide best prediction of planned procedure characteristics of interest, such as anticipated duration, potential complications, staff needs and equipment needs. The invention may provide a user-facing system that allows entry of all specifics that cannot be captured automatically. The invention may present the prediction by a user interface to a physician (e.g. an urologist).

The invention may provide a database which per case logs information comprising e.g.
stone specifics such as number, size(s), position(s), composition(s), procedure specifics such as planned and realized access path, path criticality (distance to organs and structures at risk), use duration, complications, equipment used, staff present (incl. experience level), procedure success (stone-free), patient specifics such as age, co-morbidities, BMI.

According to an embodiment, the user recommendation may comprise a recommendation on at least one of: workflow adjustment, risk stratification, possible side effects, procedural recommendations.

According to an embodiment, the procedural recommendations may be from at least one of: planned and realized access path, distance to organs, structures at risk, recommended procedure duration, recommended equipment, staff availability.

According to an embodiment, the method may further comprise providing, by the processor, a database comprising historical patient data, historical task data, and historical planning data. This may be advantageous as it may increase the reliability of the prediction model. The term database, as used herein, is to be understood broadly may relate to any digital database configured to store data in an organized manner.

According to an embodiment, the method may further comprise storing the current patient data, the current task data, the determined planning data and realized planning data in the database. This may be advantageous as it may lead to a higher prediction quality of the trained model. The prediction model may be continuously trained. The current patient data, current task data, the determined planning data and the realized task data may be automatically or manually received by the database by means of a data interface. The interface may be in case of manually receiving an HMI (i.e. user interface) configured to receive input from for example staff carried out the surgical process.

According to an embodiment, a method is provided, further comprising receiving historical patient data, historical task data, and/or historical planning data by a user interface. This may be advantageous as some specific data cannot be received automatically such as observations made by the staff. The user interface may be a tablet, PC, HMI or the like.

According to an embodiment, a method is provided comprising selecting, by utilizing the prediction model and the obtained current patient data and current task data a set of historical patient data, historical task data and historical planning data and providing the selected set. This may be advantageous as the set shows in detail a person who will carry out a surgical process, how in the past a similar case has been carried out. Hence, the selected data from the database may serve as supplementary assistance to the determined planning data.

According to an embodiment, the patient data may comprise at least one of the following: age, co-morbidities, BMI and/or wherein the historical planning data may comprise at least determined planning data and realized planning data. The term realized planning data may relate to the implemented planning data to carry out the surgical process, for example, the implemented access route to a kidney stone. The implemented access route to the kidney stone may deviate from the previously determined access route. This may also be advantageous for a practitioner as it helps him to classify the determined planning data concerning the reliability in some cases.

According to an embodiment, the patient data may comprise a CT image of the subject and/or at least a part of the patient data may be extracted from the CT image. This may be advantageous as a CT image and corresponding information may provide a more detailed information.

According to an embodiment, the task data may comprise at least information about an object being located in the subject to be treated and/or a standard access path to the object being in the subject. The standard access path may comprise for example a specific calyx in which a kidney stone is located. The information may be presented in a modified CT image that may depict the access path and the location of the kidney stone. This may reduce the complexity of the upcoming surgery.

According to an embodiment, the planning data may comprise at least one of the following: needed equipment for the surgical process, duration of the surgical process, staff required for the surgical process, risk for adverse events and an access path to an object being located in the subject to be treated. This may be advantageous in terms of planning quality.

According to an embodiment, the planning data may comprise contact information of an attending physician. This may be useful in order to receive further information in case that there are still questions remaining. The contact information may comprise address, phone number, e-mail address or the like. The contact information may be retrieved from the database for a case that is similar to the current case, wherein the case is described or defined by the planning data, patient data and task data.

According to an embodiment, the method may comprise determining an uncertainty measure for the determined planning data. The term uncertainty measure, as used herein, is to be understood broadly and may relate to an uncertainty of the determined planning data. The uncertainty measure may also be determined by using the prediction model. In other words, the uncertainty measure is a further result of the prediction model, which reveals the quality of the prediction result. The uncertainty measure may be presented in the same manner as the planning data as described above. The uncertainty measure may be on a scale between 0 and 1 or 0 and 100% or the like. The uncertainty measure may also be used to determine uncommon cases. This may be a helpful information for staff entrusted with specific case.

According to an embodiment, based on the determined uncertainty measure a case report of the surgical process may be generated and provided for further processing. For example, in case the uncertainty measure is below a threshold (e.g. 80%) a case report is generated as the current surgery indicates an uncommon case. This case report may be used for further analysis regarding the planning data, patient data and task data. This may be helpful to get a better understanding and awareness of difficult cases. The case report may comprise any available data for the specific surgery. Since the prediction model can be trained to also provide uncertainty measures to these predictions, these cannot only be shown but also used to identify uncommon cases, that is, cases whose characteristics can only be predicted with much uncertainty. This can be indicated to the user with a recommendation to publish the case report. These case reports could also be used to train young professionals.

According to an embodiment, the trained prediction model is continuously trained. In other words, the current patient data, the determined planning data and the current task data may be used to train the prediction model. This may be advantageous in terms of prediction quality and reliability.

A further aspect relates to a device for determining planning data for a surgical process of a subject, comprising means for carrying out the steps of the method described above. The means may comprise a processor, computer unit, a workstation with corresponding interfaces for providing the prediction model and obtaining patient data, task data and determining planning data. The means may comprise a screen, a dashboard, a tablet or the like for providing and presenting the determined planning data.

A last aspect relates to a computer program comprising instructions, which when the program is executed by a computer, cause the computer to carry out the method described above, and/or to a computer-readable medium comprising instructions which, when executed by a computer, cause the computer to carry out the method described above.

The computer program might be stored on a computer unit, which might also be part of an embodiment. This computer unit may be configured to perform or induce performing of the steps of the method described above. Moreover, it may be configured to operate the components of the above-described device. The computing unit can be configured to operate automatically and/or to execute the orders of a user. A computer program may be loaded into a working memory of a data processor. The data processor may thus be equipped to carry out the method according to one of the preceding embodiments. This exemplary embodiment of the invention covers both, a computer program that right from the beginning uses the invention and computer program that by means of an update turns an existing program into a program that uses invention. Further, on, the computer program might be able to provide all necessary steps to fulfil the procedure of an exemplary embodiment of the method as described above. According to a further exemplary embodiment of the present invention, a computer readable medium, such as a CD-ROM, USB stick or the like, is presented wherein the computer readable medium has a computer program stored on it which computer program is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems. However, the computer program may also be presented over a network like the World Wide Web and can be downloaded into the working memory of a data processor from such a network. According to a further exemplary embodiment of the present invention, a medium for making a computer program available for downloading is provided, which computer program is arranged to perform a method according to one of the previously described embodiments of the invention.

It is to be understood that the user recommendation on planning of a urology procedure planning, such as a PCNL procedure, may be displayed directly to the user of the imaging device (such as urology station, ultrasound device) or transmitted to a DICOM station. In some embodiments, the recommendation is incorporated in an EHR system. In some embodiments, the recommendations displayed also include recommendations on the need for consultations with additional specialists, such as interventional radiologists.

It is noted that the above embodiments may be combined with each other irrespective of the aspect involved. Accordingly, the method may be combined with structural features of the device and/or system of the other aspects and, likewise, the device and the system may be combined with features of each other, and may also be combined with features described above with regard to the method.

These and other aspects of the present invention will become apparent from and elucidated with reference to the embodiments described hereinafter.

### BRIEF DESCRIPTION OF THE DRAWINGS

Exemplary embodiments of the invention will be described in the following drawings.
Fig. 1 is a schematic view of the method for determining planning data for a surgical process of a subject in a urology procedure;
Fig. 2 shows a schematic view of a device for determining planning data for a surgical process of a subject in a urology procedure; and
Fig. 3 shows exemplary images selected by means of the method for determining planning data for a surgical process of a subject in a urology procedure.

### DETAILED DESCRIPTION OF EMBODIMENTS

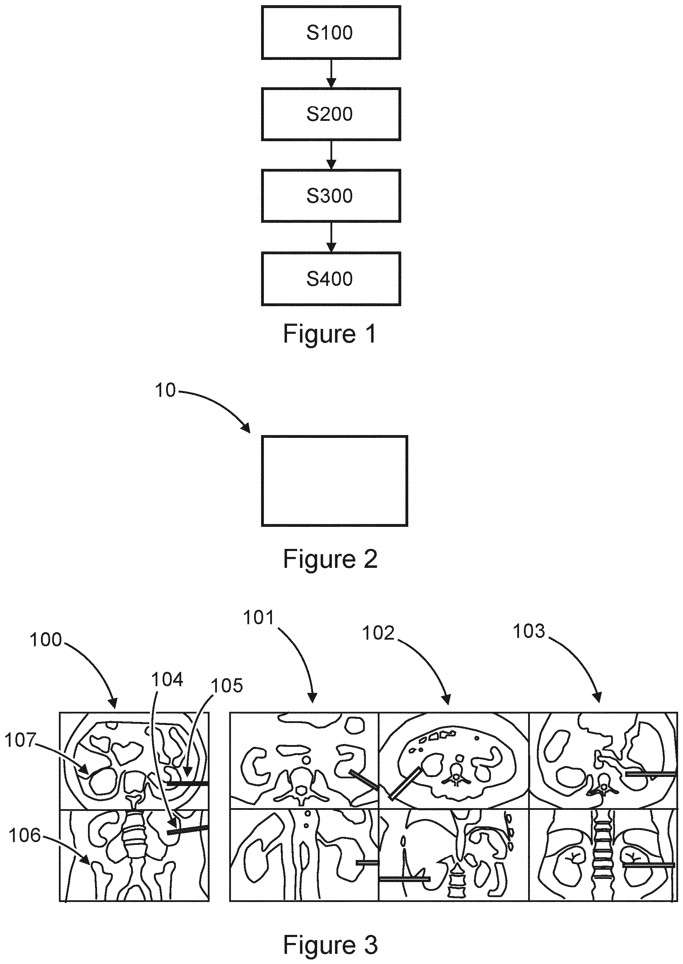

Fig. 1 is a schematic view of the method for determining planning data for a surgical process.

Step 100 comprises providing, by a processor, a prediction model trained to predict planning data based on historical patient data, historical task data, historical planning data, current patient data and current task data. In the present example, the prediction model may be a machine learning model. The surgical process may be a percutaneous nephrolithotomy (PCNL) used to remove stones from a kidney through a skin puncture. The subject may be a human in the present example.

The prediction model is a model that was trained with historical patient data, historical task data, and historical planning data. The historical planning data may comprise at least determined planning data and realized planning data.

The planning data may comprise one of the following: needed equipment for the surgical process, duration of the surgical process, staff required for the surgical process, risk for adverse events and an access path to an object being located in the subject to be treated.

The task data (historical and current) may comprise at least information about an object being located in the subject to be treated and/or a standard access path to the object being in the subject. In the present example, the task data may comprise the position, the size and/or the form of the one or more stones in the kidney. The standard access path may comprise the one or more calyx suitable to reach the stone. The standard access path may be depicted on a CT image.

The patient data may comprise at least one of the following: age, co-morbidities and BMI. The patient data may comprise a CT image of the subject and/or at least a part of the patient data is extracted from the CT image. The planning data (historical and current) may comprise contact information of an attending physician. The processor may be part of a computer unit, a workstation or a virtual machine. The processor may be a single entity or distributed on a plurality of entities.

Step 200 comprises obtaining, by the processor, current patient data and current task data. The current patient data and current task data may be obtained automatically by means of an interface between the processor and a database system (e.g. hospital database system). The current patient data and current task data may be obtained manually from user input via an HMI, wherein staff (e.g. physician) inputs current patient data and current task data.

The current patient data may comprise in the present example, that the patient has a BMI of 26.6 and an age 36. The current task data may comprise that there is one stone with an approximately round shape in a position relative to a reference point that has to be removed. The current patient data may comprise a corresponding diagnostic CT image.

Step 300 comprises inputting the at least one of the current patient data and current task data to the prediction model in order to determine planning data. The prediction model receives the current patient data and current task data as input and provides the planning data as output.

The determined planning data comprises an interpolation between the data points/sets used to train the prediction model. The determined planning data may then comprise for example that a specific tool is needed, an access path through a specific calyx is proposed and a urologist is required and that the procedure will last 85 minutes and that no complications are expected.

Step 400 comprises providing a user recommendation on planning of a urology procedure planning, such as a PCNL procedure, by the processor, based on the determined planning data. The user recommendation may be depicted in a figure, a report, a table, an adapted CT-image or the like. The user recommendation may presented on a screen or a dashboard.

Additionally, the user recommendation may comprise a recommendation on at least one of: workflow adjustment, risk stratification, possible side effects, procedural recommendations.

Additionally, the procedural recommendations may be from at least one of: planned and realized access path, distance to organs, structures at risk, recommended procedure duration, recommended equipment, staff availability.

The method may further comprise receiving historical patient data, historical task data, and/or historical planning data by a user interface. The method may further comprise selecting, by utilizing the prediction model and the obtained current patient data and current task data, a set of historical patient data, historical task data and historical planning data, and providing the selected set.

The method may further comprise determining an uncertainty measure for the determined planning data. Based on the determined uncertainty measure a case report of the surgical process may be generated and provided for further processing. The prediction model may be trained continuously with any further received data set of a current carried out surgery.

Fig. 2 shows a schematic view of a device for determining planning data for a surgical process of a subject in a urology procedure.

The device 100 comprises a processor. The processor may be a part of a computer unit. This computer unit may be configured to perform or induce performing of the steps of the method described above. In the present example, the processor is part of a workstation located in the hospital. Alternatively, the processor may also be located in a cloud application and accessed by an interface and corresponding communication unit. The device may further comprise presenting means such as a screen or dashboard. The device may further comprise one or more interfaces for data exchange.

Fig. 3 shows exemplary images selected by means of the method for determining planning data for a surgical process of a subject in a urology procedure.

Fig. 3 comprises four diagnostic CT images 100, 101, 102 and 103. Image 100 shows a diagnostic CT image of a subject to be treated in two views 106, 107 each comprising an access path 104 and 105. The diagnostic CT images 101, 102 and 103 refer to diagnostic CT images from historical cases stored in the database.

The diagnostic CT images 101, 102 and 103 show each also two views and corresponding access paths. The diagnostic CT images 101, 102 and 103 are selected by the method described above from the plurality of cases stored in the database.

The selected diagnostic CT images 101, 102 and 103 show the greatest similarity with the current task data and the current patient data. In particular, the diagnostic CT image 103 shows the best match of the three images 101, 102, and 103. This set of CT images is presented to a user in a planning phase, e.g. a physician, who has to carry out the surgery.

### LIST OF REFERENCE SIGNS:

- S100: providing a trained prediction model
- S200: obtaining current patient data and current task data
- S300: determining planning data
- S400: providing user recommendation

- 10: device
- 100, 101, 102, 103: diagnostic CT image
- 104, 105: access path
- 106, 107: different views

## Claims

1. Computer-implemented method for determining planning data for a surgical process of a subject in a urology procedure, comprising:
providing, by a processor, a prediction model trained to predict planning data based on at least one of: historical patient data, historical task data, historical planning data, current patient data and current task data (S100);
obtaining, by the processor, at least one of: current patient data and current task data (S200);
inputting the at least one of the current patient data and current task data to the prediction model in order to determine planning data (S300);
providing a user recommendation on planning of a urology procedure planning, such as a PCNL procedure, by the processor, based on the determined planning data (S400).

2. The method of claim 1, wherein the user recommendation may comprise a recommendation on at least one of: workflow adjustment, risk stratification, possible side effects, procedural recommendations.

3. The method of claim 2, wherein the procedural recommendations are from at least one of: planned and realized access path, distance to organs, structures at risk, recommended procedure duration, recommended equipment, staff availability.

4. Method according to any one of the preceding claims, further comprising receiving historical patient data, historical task data, and/or historical planning data by a user interface.

5. Method according to claim 2, further comprising selecting, by utilizing the prediction model and the obtained current patient data and current task data a set of historical patient data, historical task data and historical planning data and providing the selected set.

6. Method according to any one of the preceding claims, wherein the patient data comprises at least one of the following: age, co-morbidities, BMI and/or wherein the historical planning data comprises at least determined planning data and realized planning data.

7. Method according to any one of the preceding claims, wherein the patient data comprises a CT image (100) of the subject and/or wherein at least a part of the patient data is extracted from the CT image (100).

8. Method according to any one of the preceding claims, wherein the task data comprises at least information about an object being located in the subject to be treated and/or a standard access path (104, 105) to the object being in the subject.

9. Method according to any one of the preceding claims, wherein the planning data comprises at least one of the following: needed equipment for the surgical process, duration of the surgical process, staff required for the surgical process, risk for adverse events and an access path to an object being located in the subject to be treated.

10. Method according to any one of the preceding claims, wherein the planning data further comprises contact information of an attending physician and wherein the planning data comprises the contact information of the attending physician.

11. Method according to any one of the preceding claims, further comprising determining an uncertainty measure for the determined planning data.

12. Method according to claim 11, wherein based on the determined uncertainty measure a case report of the surgical process is selected and provided for further processing.

13. Method according to any one of the preceding claims, wherein the trained prediction model is continuously trained.

14. Device (10) for determining planning data for a surgical process of a subject, comprising means for carrying out the steps of the method according to claim 1.

15. Computer program comprising instructions, which when the program is executed by a computer, cause the computer to carry out the method according to any one of the claims 1 to 13; and/or a computer-readable medium comprising instructions which, when executed by a computer, cause the computer to carry out the method according to any one of the claims 1 to 13.
